# EUROPEAN PATENT APPLICATION

(11) **EP 3 733 158 A1**
(43) Date of publication of application: **04.11.2020**
(21) Application number: 20172687.4
(22) Date of filing: 04.05.2020
(51) Int. Cl.: A61K 8/43, A61K 8/67, A61K 31/14, A61K 31/155, A61K 31/355, A61P 17/00, A61P 17/04, A61P 17/08, A61P 17/16, A61P 43/00, A61Q 19/00

(54) **CREAM FOR DERMATOLOGIC USE FOR THE TREATMENT AND PREVENTION OF SKIN DISEASES IN ONCOLOGY**

(30) Priority: 02.05.2019 IT 201900006493
(71) Applicant: Cinnapharm S.A.S. di Tombolato Alberto Denis e C., 31030 Castello di Godego (TV) (IT)
(72) Inventor: TOMBOLATO, Alberto Denis, 31030 CASTELLO DI GODEGO (TV) (IT)
(74) Representative: Vinci, Marcello

(57) **Abstract**

The invention is a combination of substances formulated as a dermatological cream normally used for the treatment and prevention of infections in all kinds of injuries, ranging from minor skin diseases or blisters to minor burns, abrasions, excoriations, insect bites, for the prevention and treatment of folliculitis, wounds, decubitus, chapped skin, diaper dermatitis, treatment of superinfections from psoriatic diseases, and for the treatment of hypertrophic scars and keloids (resulting from surgical treatment, traumatic injuries and burns) after complete healing of the injury and with intact/closed skin surface; the innovative use of this cream is related to skin diseases in oncological patients, including the Hand-Foot Syndrome, which occurs in case of treatment with antineoplastic drugs. This combination includes the following substances: cetrimide, chlorhexidine gluconate, alpha tocopherol and co-formulants.

## Description

The present invention concerns an innovative use of a combination of substances formulated as a dermatological cream for the treatment and prevention of skin diseases in the field of oncology. The composition of the same includes cetrimide, chlorhexidine gluconate, liquid alpha tocopherol acetate Fu-Ph and co-formulants.

This combination of substances is effective in the treatment of the Hand-Foot Syndrome that develops, as an adverse reaction, following the use of antineoplastic drugs, among which mainly capecitabine, fluorouracil and doxorubicin, and less frequently cyclophosphamide, cytarabine, docetaxel, mercaptopurine, paclitaxel and vinorelbine.

This syndrome, which is called palmar-plantar erythrodysesthesia, starts with a feeling of numbness and burning at the palmar and plantar level. These initial conditions are usually followed by various pathological stages, which may give rise to: dermatosis, with erythema, edema or hyperkeratosis; skin alterations such as desquamation, blisters and bleeding; finally, more severe alterations of the skin that limit everyday activities.

Said adverse reaction, for capecitabine only, has a prevalence of 53-57% of cases, of which 11-17% in severe form. The resolution of this adverse effect can be complicated by local factors, such as marked skin dehydration and infections generated by the injury.

Skin infections may derive from exogenous contamination, due to microorganisms, or endogenous contamination.

It should not be forgotten that skin is sterile at birth and successively becomes colonized with bacterial strains that vary with age. Said bacteria, among which *Staphylococcus Aureus* and *Staphylococcus Epidermidis*, which are Gram-positive bacteria, and *Pseudomonas Aeruginosa*, which is a Gram-negative bacillus, can contribute to the development of pathogenic skin infections, in the case where the skin is affected by factors such as comorbidity, dryness and flaking. More specifically, the bacteria like Staphylococcus, which are among the most common pathogens in humans, being largely present in the skin, have several virulence factors. Among them, the following are worth mentioning: cytotoxin α, β, γ, δ and leukocidin, which are toxic for many cells, including leukocytes, erythrocytes, macrophages, platelets and fibroblasts; exfoliative toxin: serine-protease capable of breaking the intercellular bridges at the level of granular layer of the epidermis; toxic shock syndrome (TSS) toxin, which causes the loss or destruction of the endothelial cells due to the activation of a strong immune response with release of cytokines and chemical mediators and consequent tissue injury. More specifically, *Staphylococcus Epidermidis,* which is a catalase-positive, coagulase-negative, Gram-positive bacterium, may cause damage if it should change its condition from that of commensal, belonging to the normal skin microflora, to that of opportunistic pathogen in certain general conditions and in specific conditions of the patient. Another pathogenic agent that infects the skin and makes it even more difficult to resolve palmar-plantar erythrodysesthesia is *Pseudomonas Aeruginosa,* a Gram-negative bacillus that behaves as an opportunistic pathogen if skin and mucosae allow it to do so. This bacterium is resistant to many antibiotics and can mutate during therapy, giving origin to even more resistant strains. Resistance is due to mutations of the porins present in the outer membrane and to the production of several beta-lactamases that can deactivate many beta-lactamases antibiotics.

The pathogenic activities of bacteria, like those described above, can be factors capable of aggravating the "*adverse drug reaction"* that takes place in the skin following the use of capecitabine, doxorubicin and other antineoplastic drugs. The resolution or improvement of palmar-plantar erythrodysesthesia can be achieved through the accurate treatment of the infections due to microorganism that affect the skin itself.

The patent document US2017/100383 A1 concerns a spray solution whose formula contains chlorhexidine and cetylpyridinium chloride, said spray being applied to the injured skin so that it exerts its anti-infective action. There are no indications to state that said solution is effective in the prevention and treatment of the diseases deriving from the use of monoclonal therapies in oncological patients, first of all palmar-plantar erythrodysesthesia, also known as hand-foot syndrome.

The patent document EP 1787637 A2 concerns a cream for veterinary use, meaning not for human use, whose composition has a disinfectant, antiseptic, cicatrizing, anti-itch, anti-redness action. In this case, either, there are no indications regarding the applicability and effectiveness of the composition described for the treatment of palmar-plantar erythrodysesthesia related to the use of chemotherapy drugs.

The patent document WO 2006/051246 A1 concerns a cosmetic composition, also for toiletry, which is useful for the treatment of skin and hair and comprises Myrica gale oil, and in some variant formulas also vitamin E and chlorhexidine. The document highlights the antifungal, antimicrobial and anti-acne properties of Myrica gale oil, but its efficacy in the treatment of palmar-plantar erythrodysesthesia related to the use of chemotherapy drugs is neither mentioned nor demonstrated.

Finally, the patent document 2015/313896 A1 describes a composition intended to be used for the treatment of the hand-foot syndrome, but this composition does not contain cetrimide, chlorhexidine gluconate, and alpha tocopherol acetate.

The subject of the present invention is a dermatological cream containing the following substances as active principles:
- cetrimide 0.001-10 % in weight
- chlorhexidine gluconate 0.001-10 % in weight (chlorhexidine gluconate 20% solution)
- liquid alpha tocopherol acetate 0.001-10% in weight.

The dermatological cream comprises also co-formulants such as:
- cetostearyl alcohol 0.001-10% in weight
- liquid paraffin 0.001-50% in weight
- purified water 0.001-50% in weight.

The subject of the present invention is thus a dermatological cream based on cetrimide, chlorhexidine gluconate, liquid alpha tocopherol acetate FU-Ph (where FU-Ph refers to the certification of the raw material according to the reference standards of the official pharmacopoeia) and co-formulants suitable for use in the treatment of skin manifestations, in general in humans; it can be habitually used for the prevention of infections in all types of injuries, ranging from minor skin diseases or bladders to minor burns, abrasions, insect bites, for the prevention and treatment of folliculitis, wounds, decubitus, chapped skin, diaper dermatitis, for the treatment of superinfections from psoriatic diseases, and for the treatment of hypertrophic scars and keloids (resulting from surgical treatment, traumatic injuries and burns) after complete healing of the injury and with intact/closed skin surface; however, the innovative aspect in the use of the cream lies in the treatment of skin diseases in oncological patients. In particular, efficacy is expected in the following cases:
- prevention of the hand-foot syndrome in the oncological patient;
- treatment of skin manifestations related to the use of capecitabine, such as the hand-foot syndrome;
- treatment of skin manifestations related to the use of tyrosine kinase inhibitors and multikinase inhibitors, as well as of anti-EGFR antibodies, such as erythema, rash (maculopapular rash, papulopustular rash, acneiform rash), xerosis;
- treatment of skin manifestations related to the use of liposomal doxorubicin, such as palmar-plantar erythrodysesthesia.

The dermatological cream is used by applying the same to the affected area. The new compound is intended for contact with the skin and has been subjected to microbiological tests according to the applicable regulations, such as citotoxicity, skin irritation and skin sensitization tests.

To sum up, given the properties and the composition of the dermatological cream that is the subject of the present invention, a hydrated environment is created, which facilitates the tissue repair processes. Thanks to the action of the cream, an improvement is achieved in the micro environment of the injury and consequently its cicatrization is promoted by the antioxidant action of vitamin E and by the combination of active principles and co-formulants in the cream in question. Therefore, with reference to the above description, the following claims are expressed.

## Claims

1. Dermatological cream suited to be used for the prevention and treatment of palmar-plantar erythrodysesthesia related to the use of capecitabine in humans, **characterized in that** it comprises:
- cetrimide 0.001-10 % in weight;
- chlorhexidine gluconate 0.001-10 % in weight (chlorhexidine gluconate 20% solution);
- liquid alpha tocopherol acetate 0.001-10% in weight.

2. Dermatological cream suited to be used for the prevention and treatment, in humans, of palmar-plantar erythrodysesthesia, erythema, maculopapular rash, papulopustular rash, acneiform rash, itch, xerosis related to the use of tyrosine kinase inhibitors and multikinase inhibitors, as well as of anti-EGFR antibodies, **characterized in that** it comprises:
- cetrimide 0.001-10 % in weight;
- chlorhexidine gluconate 0.001-10 % in weight (chlorhexidine gluconate 20% solution);
- liquid alpha tocopherol acetate 0.001-10% in weight.

3. Dermatological cream suited to be used for the prevention and treatment of palmar-plantar erythrodysesthesia related to the use of liposomal doxorubicin in humans, **characterized in that** it comprises:
- cetrimide 0.001-10 % in weight;
- chlorhexidine gluconate 0.001-10 % in weight (chlorhexidine gluconate 20% solution);
- liquid alpha tocopherol acetate 0.001-10% in weight.

4. Dermatological cream according to any of the preceding claims, **characterized in that** said co-formulants comprise:
- cetostearyl alcohol 0.001-10% in weight;
- liquid paraffin 0.001-50% in weight;
- purified water 0.001-50% in weight.
